# EUROPEAN PATENT SPECIFICATION

(11) **EP 0 488 333 B1**
(45) Date of publication and mention of the grant of the patent: **05.03.1997**
(21) Application number: 91120458.4
(22) Date of filing: 28.11.1991
(51) Int. Cl.: A61N 5/06

(54) **An apparatus and its associated system for the chromatic excitation of quartz elements for therapeutic purposes**
Gerät und Zubehörteile für Therapie durch chromatische Anregung eines Quartzelementes
Appareil et accessoires pour la thérapie par excitation chromatique d'un élément en quartz

(30) Priority: 29.11.1990 ES 9003063
(43) Date of publication of application: 03.06.1992
(73) Proprietor: CROMO WORLD CORPORACIO, S.L., E-08008 Barcelona (ES)
(72) Inventor: Alfaro Banon, Neus, Cromo World Corporacio S.L., E-08008 Barcelona (ES); Pla Margalef,Juan Enric, Cromo World Corporacio SL, E-08008 Barcelona (ES)
(74) Representative: Kador & Partner

(56) References cited:
- DE-A- 2 943 117
- DE-A- 3 107 504
- FR-A- 2 561 515
- FR-A- 2 598 921

## Description

This invention patent refers to the field of therapeutic systems involving the differential application of chromatic impulses and the associated vibration which can be generated with quartz cristals.

DE-A-2,943,117 concerns an apparatus for phototherapy in which light of different colours is applied to a patient using a chromic generator, a control means, an amplifier, and an array of filters to produce a beam of light of a desired colour.

In particular this patent refers to a system for obtaining the excitation of the quartz elements and the apparatus required to operate the system.

Fundamentally this invention is based on the projection of colour emitted from a white luminous source, passing through colour filters and then projecting the said beam of coloured light through a quartz element which is the applicator body.

The natural mineral quartz is the ideal element for this type of application in that it is a highly piezo-electric and pyro-electric mineral having a series of inherent characteristics which are therapeutic in respect of the human body such as the capability for polarizing reactions of the nervous type and also acting as an energy transmitter which circulate in humans in the neuro-vegetative system. Further it can be applied satisfactorily to external treatment as for example in massageing. Another peculiarity renders it ideal for luminosity applications it being stable up to a temperature of 573 deg.C. such that it can withstand heat as a luminous source without change in its molecular composition or other properties.

The ideal type of quartz for purposes of this invention is the common quartz of the transparent or the translucent variety, on condition that the translucency be white.

The basis of the invention is that when a pre-determined coloured light is passed through a quartz cristal the latter acts as a means of augmenting the chromatic vibration, the luminous frequency then being emitted increased as a function of the size, shape and cristalization purity of the quartz applicator.

According to the present invention, a method for the chromatic excitation of quartz elements (1) for therapeutic purposes is claimed whereby luminous radiation of variable intensity and chromatism from a light generator (9) controlled by means of a programmed computer (6), with intermediate filtration by specific filters (10,11) of the light beam emitted by the generator is supplied to crystals of the natural mineral quartz, which operate as therapeutic applicators.

An apparatus according to the invention for the chromatic excitation of an element (1) for therapeutic purposes formed of the natural mineral quartz includes a computer (6) with its monitor (8), programmed to control through an interface (5) a chromatic generator (4) which projects a luminous beam on to said element (1), said chromatic generator (4) comprising a light generator (9), a series of infrared and ultraviolet filters (10,11) as well as colour discs and obturators (13,14) controlled by servo-mechanisms (12).

The chromatic generator (4) and quartz element (1) are advantageously remote from one another and connected by fibre optics (3). Said element (1) is preferably of transparent or translucent quartz and has polished pyramidal (2) or rounded facets.

In accordance with the principles of the present invention the illuminated quartz element may be utilized for two main types of application.
1. The capacity for irradiation of the beamed chromatic vibration.
   For this application a wide range of structures and sizes of quartz elements can be used. It employs the absorption capacity for external and general chromatism of the body, this taking the form of chromatic massage, colour absorption by visualization or by contact with the quartz itself.
2. The capacity for concentration of the augmented chromatic vibration.
   This application requires specific shapes and sizes so as to cause the concentration effect with facets of the quartz polished, having a paramidal shape with the apex facet pointed or rounded. The rounded apex permits use in point applications and in areas where the body is able to absorb chromatism. The pointed end is used exclusively for concentrated point applications as for example in the so-called chromopunture.

The principles of this invention require that the luminous projection for the above mentioned applications be from a white light.

As the luminosity is artificial, that is, not solar, it is immaterial as to whether the light emission be continuous or alternating. Neither would affect the treatment.

The intensity of the luminous projection will be a function of the utilization or the size of the quartz.

Any type of filter may be employed in order to obtain a specific colour.

For materials derived from paper aniline dyes have given the best results in test projections though these have the disadvantage of material weakness and inability to withstand temperatures in excess of 35 deg.C.

A satisfactory medium for this purpose is the water filter, diluted with aniline to obtain an excellent chromatic range and good transparency. A disadvantage is its short life and laborious preparation as well as problems of size. Water boils at 100 deg.C. affecting the filtration and with the danger of breaking its container.

Gelatines represent the ideal up to the present for the chromatic operation in this system being adaptable to any shape or size and relatively strong though being subject ot the normal wastage of this type of material.

When the luminous source cannot be or must not be in direct contact or in semi-contact with the quartz the separation can be bridged by means of a light conductor such as optical fibres or quartz fibres giving the maximum reliability of light intensity and reflection with the possibility of adapting the fibre thickness to the projection requirements.

For the best results it is advisable to enclose the fibre conductor bundle with a totally opaque material which is also flexible and thermally insulating.

The fibre conductors should not exceed 200m in length so as to avoid loss in the reflection purity.

To assist the explanation drawings are attached showing an embodiment of the system and apparatus disclosed in this patent.

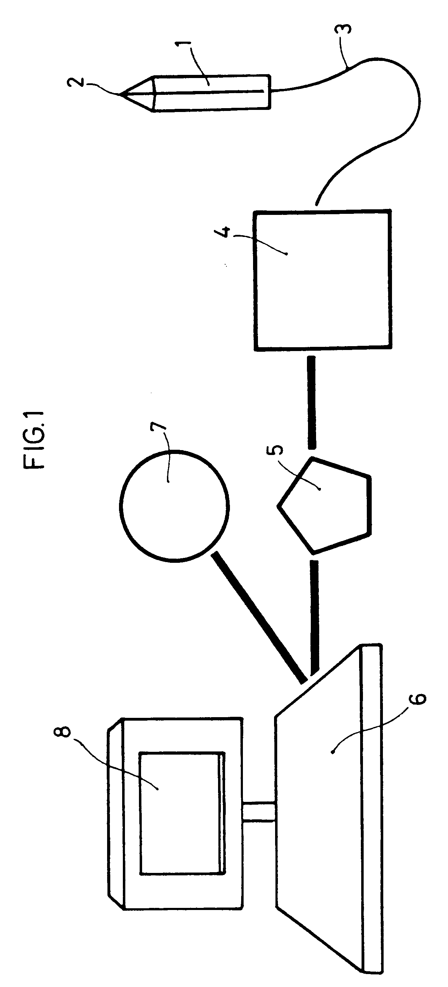

Figure 1 is a block diagram showing the system and the scheme layout of the apparatus.

Figure 2 shows in schematic form the connection between the light generator and the photo-impulsor.

As may be seen in the drawings, the system object of this patent involves the excitation of the quartz photo-impulsors -1-, of natural translucid quartz, specifically white in colour and cut to a rounded or pointed shape as shown at reference -2- in the drawings, these photo-impulsors of quartz -1- receiving a filtered luminous radiation through a light conducting.fibre cable -3-, possible optical or quartz from a chromatic generator -4- which in turn is controlled through an interface -5- by a computer -6- programmed -7-, with a control monitor -8-.

The data program contains all the instructions concerning the management, flow, control, display, and other items constituting the peripherals and also holds the library of information and data required for the application.

The interface -5- is an electronic circuit gathering the data from the computer -6-, storing them momentarily and treating it for conversion to complex and cadenced orders in interaction with the control panel -8-. It also contains the operation commands for changing colours by means of phased motors or servomotors.

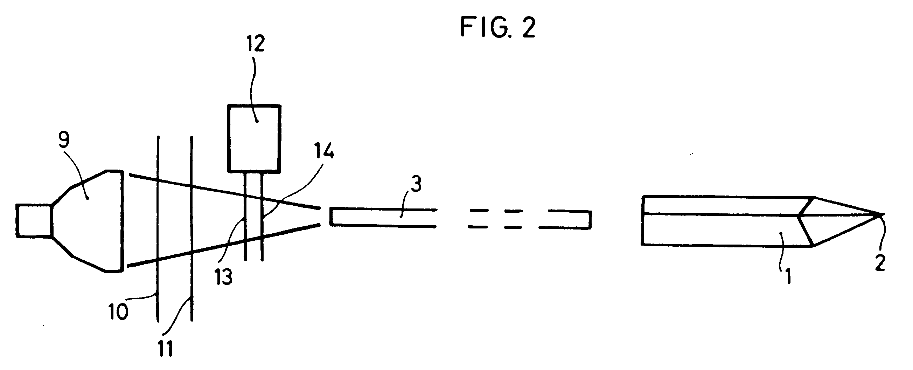

Figure 2 shows in greater detail the composition of the light generator, optic fibre bundle and the quartz cristal.

As indicated above the quartz cristal -1- with its application point -2- as shown or rounded is fed by a light beam either direct or through a fibre optic bundle conductor -3-. The light beam is emitted from a light generator -9- having a selection of filters such as -10- and -11- for infrared and ultraviolet radiation and in general for the filtration of the initial light beam as may suit the treatment. Servomechanisms -12- are interconnected with a system of colour discs and obturators -13- and -14-. By means of the components mentioned above it is possible to excite the quartz elements with the requisite luminous radiation and to apply the resulting chromatic vibrations to the appropriate parts of the human body.

## Claims

1. A method for the chromatic excitation of quartz elements (1) for therapeutic purposes, whereby luminous radiation of variable intensity and chromatism from a light generator (9) controlled by means of a programmed computer (6), with intermediate filtration by specific filters (10,11) of the light beam emitted by the generator is supplied to crystals of the natural mineral quartz, which operate as therapeutic applicators.

2. Apparatus for the chromatic excitation of an element (1) for therapeutic purposes formed of the natural mineral quartz, whereby a computer (6) with its monitor (8), programmed to control through an interface (5), a chromatic generator (4) which projects a luminous beam on to said element (1), said chromatic generator (4) comprising a light generator (9), a series of infrared and ultraviolet filters (10,11), colour discs and obturators (13,14) controlled by servo-mechanisms (12) is included.

3. Apparatus according to claim 2 characterized in that the chromatic generator (4) and quartz element (1) are remote from one another and are connected by fibre optics (3).

4. Apparatus according to claim 1 characterized in that said element (1) is of transparent or translucent quartz and has polished pyramidal (2) or rounded facets.

## Patentansprüche

1. Verfahren zur chromatischen Anregung von Quarzelementen (1) für therapeutische Zwecke, wobei Lichtstrahlung mit unterschiedlicher Intensität und chromatischer Abweichung von einem Lichtgenerator (9), der von einem programmierten Computer (6) gesteuert wird, bei Zwischenfiltration des vom Generator abgegebenen Lichtstrahles durch bestimmte Filter (10,11) natürlichen Mineralquarzkristallen zugeführt wird, die als therapeutische Anwendungseinrichtungen wirken.

2. Vorrichtung zur chromatischen Anregung eines Elementes (1) für therapeutische Zwecke, das aus natürlichem Mineralquarz hergestellt ist, wobei ein Computer (6) mit Monitor (8), der für die Steuerung über eine Schnittfläche (5) programmiert ist, ein chromatischer Generator (4), der einen Lichtstrahl auf das Element (1) richtet, wobei der chromatische Generator (4) einen Lichtgenerator (9) umfaßt, eine Reihe von IR- und UV-Filtern (10,11), Farbscheiben und Absperreinrichtungen (13,14) enthalten sind, die von Servo-mechanismen (12) gesteuert werden.

3. Vorrichtung nach Anspruch 2, dadurch gekennzeichnet, daß der chromatische Generator (4) und das Quarzelement (1) entfernt voneinander angeordnet und durch Lichtwellenleiter verbunden sind.

4. Vorrichtung nach Anspruch 1, dadurch gekennzeichnet, daß das Element (1) transparenter oder durchscheinender Quarz ist und geschliffene, pyramidenförmige (2) oder abgerundete Facetten aufweist.

## Revendications

1. Procédé d'excitation chromatique d'éléments de quartz (1) à des fins thérapeutiques, par lequel un rayonnement lumineux de chromatisme et d'intensité variables provenant d'un générateur de lumière (9) commandé au moyen d'un ordinateur programmé (6), avec une filtration intermédiaire par des filtres spécifiques (10, 11) du faisceau lumineux émis par le générateur, est fourni à des cristaux de quartz minéral naturel qui servent d'applicateurs thérapeutiques.

2. Dispositif d'excitation chromatique à des fins thérapeutiques d'un élément (1) fait de quartz minéral naturel, dans lequel il y a un ordinateur (6) et son écran de surveillance (8), programmé pour commander par l'intermédiaire d'une interface (5) un générateur chromatique (4) qui envoie un faisceau lumineux sur ledit élément (1), ledit générateur chromatique (4) comprenant un générateur de lumière (9), une série de filtres (10, 11) à infra-rouges et à ultra-violets, des disques de couleur et des obturateurs (13, 14) commandés par des servo-mécanismes (12).

3. Dispositif selon la revendication 2, caractérisé en ce que le générateur chromatique (4) et l'élément de quartz (1) sont éloignés l'un de l'autre et sont reliés par fibres optiques (3).

4. Dispositif selon la revendication 1, caractérisé en ce que ledit élément (1) est fait de quartz transparent ou translucide et comporte des facettes arrondies ou pyramidales polies (2).
